# FASCICULE DE BREVET EUROPEEN

(11) **EP 2 817 607 B1**
(45) Date de publication et mention de la délivrance du brevet: **04.12.2019**
(21) Numéro de dépôt: 13705493.8
(22) Date de dépôt: 22.02.2013
(51) Int. Cl.: G01N 3/30, G01N 3/32, G01N 3/42, G01N 33/24

(54) **TETE DE MESURE DESTINEE A EQUIPER UN PENETROMETRE DYNAMIQUE ET PROCEDE DE MESURE A L'AIDE D'UNE TELLE TETE DE MESURE**
MESSKOPF FÜR DYNAMISCHES PENETROMETER UND MESSVERFAHREN UNTER VERWENDUNG EINES SOLCHEN MESSKOPFES
MEASURING HEAD INTENDED TO BE FITTED TO A DYNAMIC PENETROMETER AND METHOD OF MEASUREMENT USING SUCH A MEASURING HEAD

(30) Priorité: 23.02.2012 FR 1251660
(43) Date de publication de la demande: 31.12.2014
(73) Titulaire: Sol Solution, 63200 Riom (FR)
(72) Inventeur: GOURVES, Roland, F-63200 Marsat (FR); BENZ NAVARRETE, Miguel, F-63000 Clermont Ferrand (FR)
(74) Mandataire: Lavoix
(86) Numéro de dépôt international: PCT/EP2013/053573
(87) Numéro de publication internationale: WO 2013/124426

(56) Documents cités:
- WO-A1-03/056302
- FR-A1- 2 732 246
- FR-A1- 2 817 344
- FR-A1- 2 938 276
- US-A- 4 116 041
- US-A- 4 332 160

## Description

La présente invention concerne une tête de mesure destinée à équiper un pénétromètre dynamique ainsi qu'un procédé de mesure à l'aide d'une telle tête de mesure.

Un pénétromètre dynamique est un appareil permettant de mesurer, in situ, les caractéristiques mécaniques des sols sans qu'il soit nécessaire de prélever un échantillon pour une étude en laboratoire.

Un pénétromètre comprend un train de tiges métalliques. L'extrémité libre d'une tige terminale est pourvue d'une pointe assurant la pénétration dans le sol d'une partie du train de tiges lorsqu'on exerce un choc sur l'extrémité libre de l'autre tige terminale du train de tiges. Ce choc est transmis au train de tiges par l'intermédiaire d'une tête de battage.

Un tel pénétromètre dynamique est décrit dans FR-A-2 817 344 et commercialisé par la société SOL SOLUTION. Ce pénétromètre permet de mesurer la compacité d'un sol et comporte une tête de battage, une tige centrale, une sonde conique et un train de tiges reliant la tête de battage à la sonde conique. Le fonctionnement du pénétromètre repose sur le principe consistant à fournir une énergie à la tête de battage, notamment par un coup de marteau. Cette énergie est alors transmise par la tige centrale au train de tiges pliées à la sonde conique. Celle-ci s'enfonce alors dans le sol d'une profondeur dépendant de la densité du sol. Connaissant l'énergie fournie au pénétromètre, la valeur du déplacement de la sonde et la section de la sonde, il est alors possible de déterminer le taux de compacité du sol. Les deux dernières caractéristiques sont facilement mesurables et l'énergie transmise au pénétromètre est mesurée par l'intermédiaire d'un capteur de type piézoélectrique dont la déformation génère un signal électrique proportionnel à l'intensité du choc. On peut également utiliser, pour mesurer l'énergie fournie au pénétromètre des jauges de contrainte montées en pont de Wheatstone. Les jauges de contrainte sont donc mises sous tension et leur résistance électrique varient en fonction de la déformation de la jauge. Le signal électrique, appliqué aux jauges de contrainte, varie donc proportionnellement à l'intensité du choc et permet de déduire la valeur de l'énergie transmise à la tête de battage. Dans ce document de l'art antérieur, on ne se préoccupe pas de mesurer l'onde transmise lors du choc et encore moins de filtrer une telle onde transmise.

L'utilisation d'un tel pénétromètre est limitée. En effet, le choc est, généralement, effectué manuellement à l'aide d'un marteau. De ce fait, la mesure des caractéristiques des sols s'effectue sur une profondeur restreinte, typiquement entre 0 et 7 m.

De plus, la mesure de l'énergie fournie au pénétromètre manque de précision et l'utilisation d'un matériau piézoélectrique tel que le quartz augmente le coût du pénétromètre.

WO03056302 A1 divulgue une sonde de sol ou de neige contenant une cellule de mesure dans la tête de sonde, ainsi qu'un accéléromètre de sorte qu'un profil de la force verticale de la neige ou du sol puisse être établi. La résistance de pénétration est mesurée au moyen de la cellule de mesure qui contient un polymère faiblement thermodurcissable choisi pour son aptitude à se comporter comme un fluide non compressible.

FR2817344 A1 met en œuvre un pénétromètre comprenant une tête de battage réalisée dans un matériau à haut module d'élasticité, une sonde conique et au moins une tige reliant la tête de battage à la sonde conique, consistant à transmettre une énergie à la tête de battage par un choc élastique.

FR2732246 A1 se rapporte à un générateur d'ondes de choc destiné à engendrer une onde de choc dans une structure en vue d'en étudier les caractéristiques. C'est à ces inconvénients qu'entend plus particulièrement remédier l'invention en proposant une tête de mesure destinée à équiper un pénétromètre dynamique permettant la détermination, de manière fiable, des caractéristiques du sol en optimisant la collecte et le traitement des signaux fournis par un pénétromètre, cela pour des épaisseurs de sol allant jusqu'à 15 m.

A cet effet, l'invention a pour objet une tête de mesure telle que définie à la revendication 1.

La présence d'un organe d'absorption propre à filtrer l'onde de choc permet une collecte optimisée des signaux tout en limitant le traitement de ces derniers, les signaux subissant ainsi une première filtration.

Des aspects avantageux mais non obligatoires de l'invention sont spécifiés aux revendications dépendantes 2 à 9.

L'invention concerne également un procédé de mesure tel que défini à la revendication 10.

L'invention sera mieux comprise et d'autres avantages de celle-ci apparaîtront plus clairement à la lecture de la description qui va suivre d'une tête de mesure réalisée conformément à l'invention, donnée uniquement à titre d'exemple et faite en se référant aux dessins annexés dans lesquels :
- la figure 1 est une vue de côté d'une tête de mesure conforme à l'invention,
- la figure 2 est une coupe longitudinale, à la même échelle, selon le plan II de la tête de mesure de la figure 1,
- la figure 3 est une vue simplifiée, de côté et à une autre échelle, de la tête de mesure de la figure 1 reliée à un train de tiges d'un pénétromètre, ce train de tiges étant partiellement représenté,
- la figure 4 est une vue de côté, simplifiée, d'un premier type de pénétromètre équipé de la tête de mesure de la figure 1, un marteau étant représenté en position avant un choc sur la tête de battage, le sol étant schématiquement illustré,
- la figure 5 est une vue en perspective, simplifiée et à plus petite échelle, d'un autre type de pénétromètre équipé de la tête de mesure de la figure 1,
- la figure 6 est un diagramme illustrant le procédé de traitement des signaux collectés par la tête de mesure et
- la figure 7 est une courbe simplifiée des signaux collectés par une telle tête de mesure représentatifs, une fois traités, du comportement d'un sol.

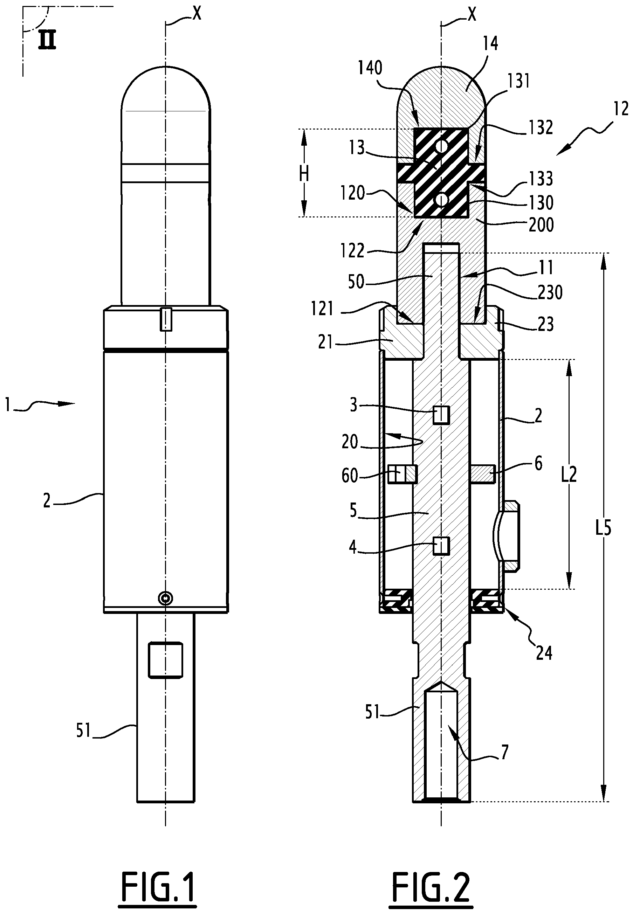

Comme cela apparait aux figures 1 et 2, la tête de mesure 1 comprend un corps principal 2 cylindrique à base circulaire. Ce corps 2, creux, est réalisé en acier. En variante, il est réalisé en un autre matériau rigide.

Au moins un, avantageusement deux capteurs de déformation 3, 4, schématiquement illustrés à la figure 2, sont montés sur une tige centrale 5 insérée longitudinalement dans le corps creux 2 de la tête de mesure 1. La tige centrale 5 est pleine et cylindrique à base circulaire. Son axe longitudinal X principal est confondu avec l'axe longitudinal principal du corps 2 lorsqu'elle est insérée dans ce dernier.

Les deux capteurs de déformation 3, 4, également dénommés jauges de contrainte, sont alignés selon l'axe X et disposés l'un par rapport à l'autre à une distance comprise entre 10% et 50 % de la longueur L2 utile du corps principal 2.

Avantageusement, comme représenté à la figure 2, un accéléromètre 6 est également disposé sur la tige 5 entre les deux jauges de contrainte 3, 4. La partie de référence 60 de l'accéléromètre 6 est fixée sur la paroi interne 20 du corps creux 2.

La tige 5 a une longueur L5 telle qu'elle dépasse du corps principal 2 par ces deux extrémités 50, 51. Les extrémités 50, 51 s'étendent au-delà du corps 2 en traversant des joints 21, 22 qui ferment les extrémités 23, 24 du corps 2. Les joints 21, 22 assurent, d'une part, l'étanchéité par rapport aux poussières et à l'eau de l'intérieur du corps 2 et, d'autre part, permettent de limiter les perturbations électromagnétiques au niveau de la tête de mesure 1, en isolant les éléments électriques et électroniques, par exemple les capteurs 3, 4 et l'accéléromètre 6, insérés dans le corps principal 2. Le corps 2 participe également, par la nature de son matériau constitutif, à la protection électromagnétique des éléments qu'il contient.

Des sorties, non représentées, permettant de connecter les différents capteurs à un module de calcul, sont également prévues sur le corps principal 2 de la tête de mesure 1.

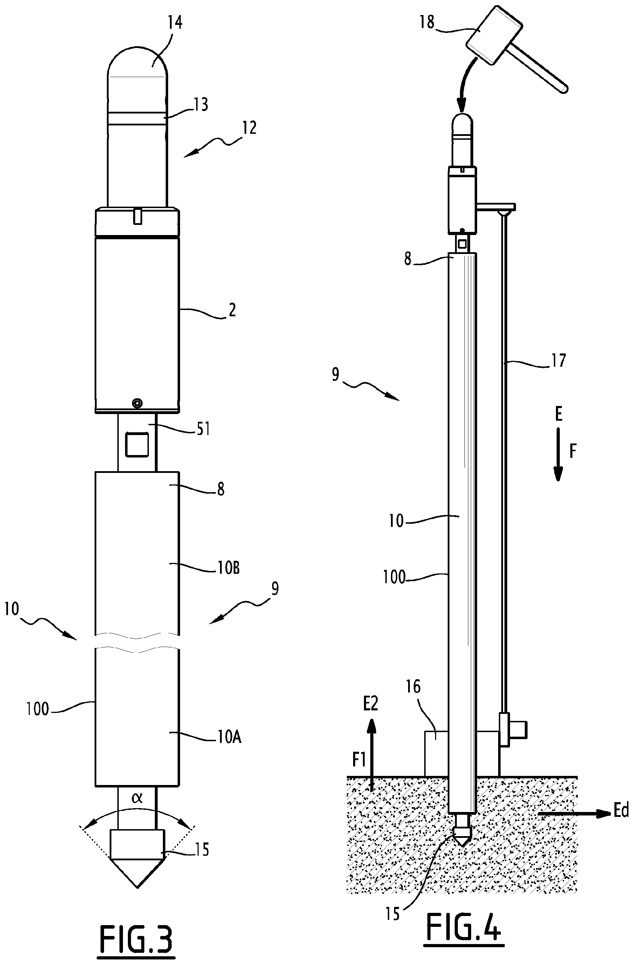

L'extrémité 51, dite basse en regardant la figure 2, de la tige 5 forme l'extrémité de la tête de mesure. Elle comprend un trou borgne 7. Le trou 7 est réalisé longitudinalement dans l'extrémité 51 et il est centré sur l'axe X de la tige 5. Le trou 7 permet la fixation de la tête de mesure 1, par un emboîtement de type mâle/femelle, connu en soi, sur une extrémité 8 d'un train 9 de tiges 10, comme illustré à la figure 3. Le train 9 de tiges 10 s'étend également selon l'axe X de la tige centrale 5 et l'extrémité 51 est, en configuration d'utilisation du pénétromètre, orientée vers le sol. Cette fixation peut être définitive. Dans ce cas, la première tige 10 du train 9 est, par exemple, soudée à la tête de mesure, le trou 7 étant adapté à une telle fixation. En variante, la fixation est réalisée de manière amovible, par exemple par vissage, le trou 7 étant alors taraudé. Une fixation amovible permet de monter une telle tête de mesure 1 « en seconde monte » sur un pénétromètre pourvu d'un train 9 de tiges 10. Plus généralement, l'extrémité 51 de la tige 5 est adaptée pour coopérer mécaniquement avec le train 9 de tiges 10.

L'extrémité supérieure 50 de la tige 5, en regardant la figure 2, est, de manière similaire à l'extrémité inférieure 51, située hors du corps principal 2 au-delà du joint 21. L'extrémité 50 est insérée dans un trou borgne 11 ménagé dans le corps principal 200 d'une tête de battage 12.

Plus généralement, l'extrémité 50 de la tige 5 est adaptée pour coopérer mécaniquement avec la tête de battage 12. Le trou 11 est réalisé en position centrale, selon une direction parallèle à un axe longitudinal de la tête de battage 12. Le corps 200 de la tête de battage 12 est en forme de cylindre allongé à base circulaire. Ses dimensions sont adaptées pour que, lorsque l'extrémité 50 est dans le trou 11, l'axe longitudinal de la tête de battage 12 soit aligné avec l'axe X du corps 2. Par conséquent, la tête de battage 12, la tige centrale 5 et le train 9 de tiges 10 sont tous alignés selon l'axe X.

Du haut vers le bas à la figure 1, on retrouve donc la tête de battage 12, puis la tige centrale 5 et enfin le train 9 de tiges 10.

Le trou 11 est ménagé dans une extrémité 121 du corps 200 reçue dans un lamage 230 réalisé dans le joint 21. L'extrémité libre 120 opposée à l'extrémité 121, est adaptée pour recevoir un organe 13 d'absorption comportant un matériau adapté pour assurer une filtration des ondes transmises au train 9 de tiges 10. Ce matériau est, avantageusement, un polymère à haut impact ou du polyéthylène à haute densité. En variante, il s'agit d'un autre matériau, par exemple du caoutchouc. Cet organe 13 est configuré en un disque plein coiffant l'extrémité 120. L'organe 13 comprend, en position centrale, deux reliefs 130, 131 disposés en regard l'un de l'autre sur deux faces 132, 133 opposées de l'organe 13 et perpendiculaires à ces faces. En variante, l'organe 13 est configuré en anneau, en double bague, en disque plein sans relief ou en toute autre forme adaptée à la configuration de la tête de mesure et/ou au filtrage souhaité.

Par ailleurs, le matériau constitutif de l'organe 13 et/ou sa configuration géométrique participent à la protection électromagnétique de la tête de mesure 1.

Le relief 130 est inséré dans un logement 122 ménagé dans l'extrémité 120. Le relief 131 est inséré dans un logement 140 ménagé dans une partie terminale 14 de la tête de battage 12, pleine et configurée sensiblement en demi-lune. Cette partie terminale 14 forme la partie de la tête de battage 12 destinée à recevoir le choc. Celui-ci est transmis, via l'organe 13 et la tige 5, à l'ensemble du train 9 de tiges 10 du pénétromètre. L'organe d'absorption 13 est donc intercalé, selon l'axe X, entre la partie terminale 14 de la tête de battage 12 et l'extrémité 51 de la tige centrale 5. Les capteurs 3 et 4 étant disposés sur la tige centrale 5, ils sont donc positionnés entre l'organe d'absorption 13 et l'extrémité 51 de la tige 5. La hauteur H de l'organe d'absorption 13 est avantageusement comprise entre 10 et 100 mm, cette hauteur étant prise entre les reliefs 130 et 131 inclus. La densité de l'organe 13, de facto la densité du matériau constitutif de l'organe 13 est comprise entre ¼ et ¾ de la densité de l'acier. Avantageusement, la densité de l'organe 13 est comprise entre 1/3 et ½ de la densité de l'acier et, de manière préférée, voisine de 1/3 de la densité de l'acier.

L'extrémité 50 de la tige 5 est insérée dans le trou borgne 11 du corps 200 de la tête de battage 12 avec un jeu. En d'autres termes, l'extrémité 50 n'est pas en contact avec le fond du trou borgne 11. Ceci participe également à l'absorption lors d'un choc exercé sur la partie 14.

La configuration de la tête 12, en particulier de la partie 14 et de l'organe 13, permet de réaliser un choc plastique et de générer une onde, sinusoïdale ou non, plus large que celle habituellement générée avec un pénétromètre de l'état de l'art. En d'autres termes, l'organe 13 absorbe certaines fréquences de l'onde dite descendante, c'est-à-dire de l'onde générée par le choc et dirigée vers le train 9 de tiges 10.

La présence de l'organe 13 permet ainsi de générer un choc et une onde de choc de type donné. Elle participe également à la protection de la tête de mesure 1 quant aux perturbations électromagnétiques.

La figure 3 illustre un train 9 de tiges 10 équipée de la tête de mesure 1. L'extrémité basse 7 de la tête de mesure 1 est reliée à une tige terminale 10 en acier, de manière définitive ou amovible, d'un train 9 de tiges 10. Le train 9 a une longueur définie par le nombre de tiges 10 et/ou la longueur de chaque tige 10, pour le type d'essais à réaliser. En général avec un pénétromètre dit à main, c'est à dire un pénétromètre dynamique à énergie variable dont la tête de battage est destinée à recevoir un choc variable exercé manuellement, cette longueur est comprise entre 0,25 m et 1 m.

La tige terminale 10A, située en position basse en regardant la figure 3, est équipée d'une pointe 15. La géométrie de cette pointe 15, également en acier, est adaptée pour que celle-ci soit conique avec un angle α au sommet au minimum de 60° et, de manière préférée, voisin de 90°. La partie active d'un pénétromètre dynamique à énergie variable comprend donc un train 9 de tiges 10 dont une tige terminale 10A comprend une pointe 15 et une autre tige terminale 10 B une tête de mesure 1.

Un tel pénétromètre dit, à main, est illustré à la figure 4. Ce type de pénétromètre est aisé à transporter. Il est représenté en configuration active, posé verticalement et perpendiculairement au sol par l'intermédiaire d'un guide 16, configuré en plaque percée, permettant le positionnement précis de la pointe 15 avant son enfoncement dans le sol.

Aux figures 3 et 4, une gaine 100 entoure le train 9 de tiges 10. Cette gaine 100 évite le contact direct entre le sol et les tiges 10. Ainsi, seule, la pointe 15 est en contact avec le sol, ce dernier n'exerçant ainsi aucun frottement sur les tiges 10 susceptible de fausser les mesures. Dans un autre mode de réalisation, selon la nature du sol, le pénétromètre est dépourvu de gaine 100.

L'extrémité haute 8 de la tige 10B haute du train 9 de tiges est pourvue de la tête de mesure 1 qui est reliée au guide 16 par une courroie 17 permettant de mesurer la distance parcourue par la pointe 15 lors de son enfoncement lorsque l'on porte un choc sur la partie arrondie 14 de la tête de battage 12. Lors de ce choc, effectué à l'aide d'un marteau 18 dont on connaît certaines caractéristiques, on réalise un choc plastique sur la partie 14. Lors de ce choc, la partie 14 transmet à la tige 5 et à la pointe 15, via le train 9 de tiges, une énergie E de haut en bas, selon une flèche F, permettant d'enfoncer la pointe 15 du pénétromètre dans le sol. Une partie Ed de cette énergie E est dissipée dans le sol, en fonction de la nature et des caractéristiques de ce dernier. Une autre partie Er de l'énergie E est réfléchie et transmise par la pointe 15 en sens inverse, c'est-à-dire de bas en haut selon la flèche F1, vers la tête de mesure 1 par le train 9 de tiges 10.

La réflexion de l'énergie Er s'effectue de manière similaire à la transmission de l'énergie E, du fait des propriétés homogènes des tiges 10 tout le long du train 9 et de la tête de mesure 1, ces différents éléments étant réalisés dans un même matériau, l'acier.

La présence de l'organe 13 permet de générer des ondes descendantes plus larges que celles générés en absence de l'organe 13, cela tout en filtrant certaines fréquences correspondant à des ondes dites parasites.

Les ondes descendantes sont, au moins partiellement, réfléchies en direction de la tête 1 à partir de la pointe 15. Ces ondes sont représentatives du comportement du pénétromètre entre la pointe 15 et le sol et entre la portion de la tige terminale 10A enfoncée dans le sol et le sol, sur la portion de tige 10A insérée dans le sol. En d'autres termes, ces ondes sont porteuses de nombreuses informations relatives au comportement mécanique du sol.

Il est donc intéressant de traiter de telles ondes afin de déterminer des caractéristiques du sol qui jusqu'à lors ne pouvaient être déterminées ou du moins ne pouvaient l'être de manière fiable et répétitive avec un pénétromètre de l'état de l'art.

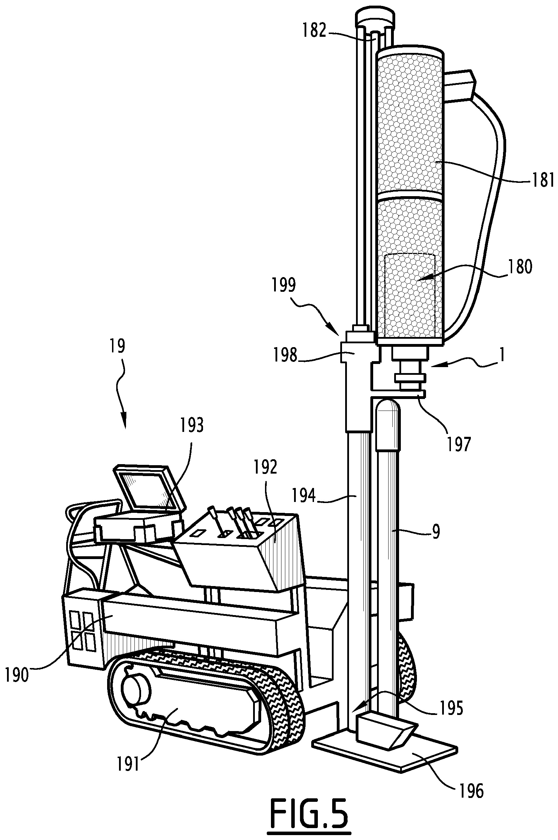

La figure 5 illustre un autre mode de réalisation de l'invention avec un pénétromètre dit lourd. Il s'agit d'un pénétromètre 19 dynamique à énergie constante, motorisé, automoteur, propre à réaliser des essais sur des profondeurs importantes du sol, typiquement au-delà de 5 m, ces essais pouvant être effectués jusqu'à des profondeurs voisines de 15 m.

Ce type de pénétromètre 19 comprend un châssis 190 pourvu des organes assurant le déplacement du pénétromètre, en l'espèce des chenilles 191. Ce châssis 190, outre un module 192 de commande du pénétromètre, comprend également un module 193 de collecte et de traitement des signaux collectés par la tête de mesure 1.

Celle-ci, comme précédemment, est disposée à l'extrémité d'un train 9 de tiges 10, guidé le long d'un mât 194 maintenu en position verticale lors des essais.

L'extrémité inférieure 195 du mât 194 est en appui sur une plaque de guidage 196 assurant le guidage de la pointe 15 dans le sol.

La tête de mesure 1 est disposée en décalée par rapport à l'axe longitudinal X du train 9 de tiges 10. Elle est reliée au train 9 par une plaque 197 fixée radialement sur la paroi externe d'un manchon 198. Ce manchon 198 est monté coulissant sur le mât 194, à partir de l'extrémité 199 de ce dernier opposée à l'extrémité 195. Une masse 180 est insérée dans une gaine 181 de guidage disposée parallèlement au mât 194. La masse 180 est mise en action par un vérin 182 inséré dans l'extrémité 199 du mât 194. La masse 180 est lâchée d'une hauteur déterminée, dans la gaine 181, avant de frapper la tête de mesure 1.

Ainsi, avec un tel pénétromètre lourd 19, il est possible de qualifier aisément l'énergie transmise lors du choc puisque la masse et la vitesse de chute de la masse 180 sont connues et définies avant chaque série de mesures. Comme précédemment, la tête de mesure 1 peut équiper de manière définitive ou amovible un tel pénétromètre 19.

Il est à noter que, dans le mode de réalisation préféré, on utilise deux jauges de déformation 3, 4 plutôt qu'une ce qui permet, à l'aide des deux mesures de déformation et de la détermination de la distance d'enfoncement de déterminer, par le calcul, l'accélération sans nécessiter la présence d'un accéléromètre 6. Ce dernier peut néanmoins, comme illustré à la figure 2, être prévu dans la tête de mesure 1, pour former un dispositif de mesure de secours en cas de défaillance d'une des jauges 3 ou 4 ou pour réaliser une mesure redondante de l'accélération.

La tête de mesure 1, qu'elle soit fixée sur un pénétromètre du type de la figure 4 ou de la figure 5, permet de collecter différents signaux. Un premier groupe de signaux comprend des données relatives à la déformation du train 9 de tiges 10 lors de la transmission de l'onde de choc de la tête de battage 12 à la pointe 15. Ces signaux sont représentatifs de la force résistante Fp(t) de la pointe. La mesure, soit directe par l'accéléromètre 6 soit par calcul, de l'accélération a(t) permet de déterminer la vitesse d'enfoncement Vp(t) de la pointe 15 ainsi que la valeur de l'enfoncement Sp(t) de la pointe 15 dans le sol. Il est ainsi possible de déterminer la résistance du sol au niveau de la pointe 15, par différence entre l'énergie EFdp(t) transmise au sol par la pointe 15 et l'énergie réfléchie par la pointe 15 .

L'énergie EFdp(t), sous forme d'une onde de compression, est transmise depuis la tête de battage 12 à la pointe 15 puis partiellement réfléchie vers la source, c'est-à-dire vers la tête de mesure 1 alors qu'une partie est absorbée par la pointe et le sol lors de l'enfoncement du pénétromètre. Lorsque cette onde réfléchie arrive sur la tête de mesure 1, elle est une nouvelle fois réfléchie, cette fois en direction de la pointe 15. Ce phénomène de yoyo de l'onde se poursuit en diminuant d'intensité à chaque réflexion par la pointe 15 ou la tête de mesure 1, jusqu'à ne plus être perceptible.

L'ensemble de ces signaux sont collectés en temps réel, à chaque passage de l'onde au niveau de la tête de mesure 1. La mesure étant effectuée environ toutes les deux microsecondes, ces signaux représentent, sur une période d'une mesure, une masse d'informations importante.

Ces informations sont représentatives du comportement mécanique du sol. En particulier, de la plasticité, de l'élasticité et de l'amortissement du sol.

Pour effectuer la détermination de ces caractéristiques à partir de l'ensemble des signaux collectés par une tête de mesure 1, qu'elle soit montée sur un pénétromètre dynamique à énergie variable ou constante tels qu'illustrés aux figures 4 et 5, il convient d'effectuer une discrimination entre les signaux représentatifs des caractéristiques du sol et les signaux parasites. Ceci est possible, en partie, grâce à l'organe d'absorption 13 et à la configuration géométrique de la partie 14 de la tête de battage 12. En d'autres termes, une filtration mécanique de certaines fréquences de l'onde de choc est réalisée par la tête de mesure 1.

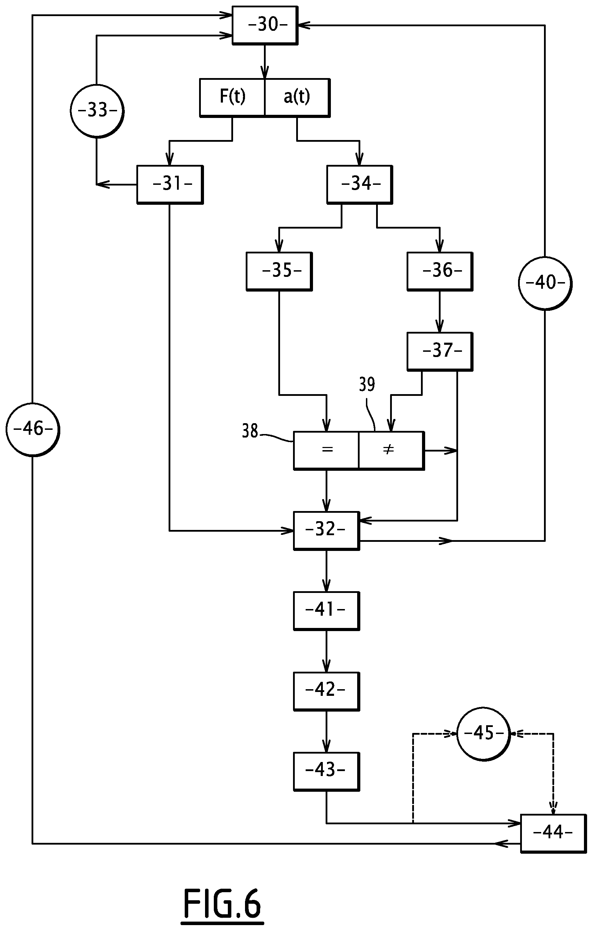

Cette filtration mécanique est complétée par un procédé de mesure des données collectées par la tête de mesure 1 illustré à la figure 6. Ici, l'expression « procédé de mesure » doit être comprise comme désignant la collecte des données par la tête de mesure 1 ainsi que le traitement et l'interprétation de ces données, étant entendu que le terme signal est à considérer comme synonyme de données.

Le procédé de mesure permet, entre autres, de traiter les signaux afin d'améliorer la lisibilité de ces derniers, en complément de l'action de l'organe 13 puis, à partir de ces signaux traités, de les interpréter et de déterminer les caractéristiques du sol par exemple, par comparaison pour certaines.

La première étape 30 consiste, à l'aide de la tête de mesure 1, à acquérir et conditionner les signaux fournis par les capteurs 3, 4. On obtient des signaux F(t) et a(t). Ces signaux subissent ensuite une série de traitements effectués par un module de calcul 193 et différents selon signal F(t) ou a(t).

Le signal de force résistante F(t) subit, lors d'une étape 31, une filtration à 50Hz afin d'éliminer les bruits électriques et un lissage pour élever la ligne de base. On obtient, à l'étape 32, un signal F(t) corrigé. Si à l'issue de l'étape 31 le signal n'est pas corrigé ou corrigeable car trop faible, il convient d'acquérir un nouveau signal F(t), comme indiquée par la flèche 33, en refaisant l'acquisition de nouveaux signaux lors d'une autre étape 30. Le signal d'accélération a(t) subit, lors d'une étape 34 un lissage et un filtrage à 50 Hz pour le bruit de fond, suivi d'une correction, au moins du premier degré, de la ligne de base.

On applique ensuite au signal a(t) un traitement d'intégration temporelle 35 et un traitement d'intégration fréquentielle 36, par transformé de Fourier rapide, suivie d'étapes 37 de filtration.

Si les valeurs 38, 39 du signal a(t) obtenues respectivement à l'issue des étapes 35 et 37 sont identiques, on obtient, à l'étape 32 un signal corrigé a(t).

Si les valeurs 38, 39 sont différentes, on privilégie la valeur 39 obtenue à l'étape 37 et cette valeur est considérée comme étant le signal a(t) corrigé à l'étape 32.

Si les valeurs de a(t) sont visiblement erronées ou trop faibles, on refait une série de mesures comme indiquée par la flèche 40.

Sur les signaux corrigés F(t) et a(t) obtenus à l'étape 32, on effectue un découplage des ondes montante et descendante lors d'une étape 41. Cette étape est suivie d'une étape 42 de détermination de la vitesse de propagation de l'onde de compression dans le sol. Il s'agit de déterminer la polaire de choc et ensuite de la célérité. Cette valeur est caractéristique du comportement du sol sous l'effet d'une onde de compression.

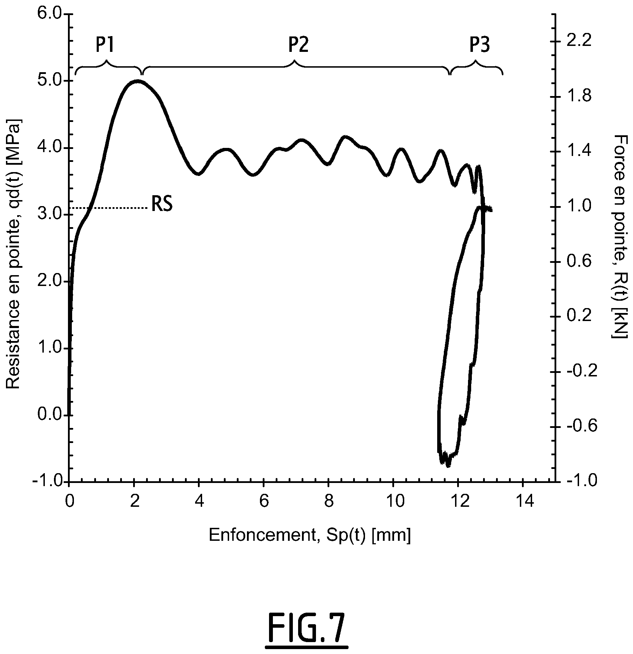

Ces corrections permettent, lors d'une étape 43, de reconstruire les signaux de résistance en pointe qd(t) en MPa, de force en pointe R(t) en kN et d'enfoncement de la pointe Sp(t) en mm et de les représenter graphiquement. Un exemple d'une telle courbe est donné à la figure 7.

Ces signaux, corrigés et exploitables sont soit affichés et comparés, lors d'une étape 44, avec des signaux de sols connus répertoriés dans une base données, soit stockés pour un traitement ultérieur lors d'une étape 45. Il est à noter que le stockage 45 intervient également après l'étape 44.

A chaque campagne de mesures, les signaux collectés à l'étape 43 viennent, par incrémentation, enrichir la base de données.

Parallèlement au stockage et/ou à la comparaison avec les valeurs connues, une autre série de mesures est réalisée, comme schématiquement illustrée par la flèche 46.

La figure 7 illustre les caractéristiques déterminées à l'étape 43 à l'issue du procédé de mesure des signaux collectés. En l'espèce, il s'agit de l'illustration du comportement d'un sol où, en fonction de l'enfoncement de la pointe en mm, sont représentées la résistance à la pointe qd(t) et également la force en pointe en R(t).

Une première partie P1 de la courbe est dite dynamique. Elle correspond à l'impact sur la pointe 15, c'est-à-dire à l'enfoncement du pénétromètre dans le sol. Cette partie P1 caractérise l'élasticité du sol.

Puis une seconde partie P2 correspond à l'amortissement du choc c'est-à-dire au ralentissement de la pénétration de la pointe dans le sol. La partie P2 dure sensiblement pendant toute la durée de l'enfoncement. Il s'agit d'une caractérisation de la plasticité. L'analyse de cette partie P2 permet également d'obtenir des informations sur la granulométrie du sol, par exemple par une analyse du signal.

La dernière partie P3 est une boucle correspondant à un cycle de chargement et rechargement. Il s'agit d'une caractérisation de l'élasticité « pure », c'est-à-dire l'élasticité due aux seules vibrations de la pointe 15 dans le sol, sans interaction de la viscosité du sol.

Il est à noter que la figure 7 est une représentation simplifiée, pour faciliter la lecture de la courbe.

En comparant les caractéristiques obtenues avec les caractéristiques connues et stockées dans une base de données, on peut associer les caractéristiques de ce sol à sa nature et/ou sa composition et/ou sa granulométrie.

La tête de mesure 1 permet de déterminer ensemble, c'est-à-dire lors d'une série de mesures, ces caractéristiques et cela lors de chaque impact. Par traitement du signal et modélisation, les signaux ainsi collectés permettent d'obtenir de nombreuses informations sur les propriétés mécaniques du sol. On peut déterminer, par exemple, la résistance statique ou résistance ultime Rs du sol. D'autres caractéristiques sont susceptibles d'être déterminées par une telle tête de mesure et un tel procédé.

## Revendications

1. Tête de mesure (1), destinée à équiper un pénétromètre dynamique (19) et adaptée pour être reliée à un train (9) de tiges (10) équipé d'une pointe (15) terminale, cette tête de mesure (1) comprenant :
- une tête de battage (12) destinée à recevoir un choc à transmettre, via le reste de la tête de mesure (1), au train (9) de tiges (10), et
- une tige centrale (5) de transmission de choc de la tête de battage au train de tiges, laquelle tige centrale présente une première extrémité (50), qui est tournée vers la tête de battage, et une seconde extrémité (51), qui est opposée à la première extrémité et qui est adaptée pour coopérer avec le train de tiges, et laquelle tige centrale est pourvue d'au moins un capteur de déformation (3, 4),
**caractérisée en ce qu'**elle comprend au moins un organe d'absorption (13), qui est intercalé entre une partie terminale de réception de choc (14) de la tête de battage (12) et la seconde extrémité (51) de la tige centrale (5) et qui est adapté pour filtrer l'onde transmise au train de tiges lorsque la partie terminale de la tête de battage reçoit un choc.

2. Tête selon la revendication 1, **caractérisée en ce que** l'organe d'absorption (13) est réalisé en un matériau comprenant un polymère à haut impact ou du polyéthylène à haute densité.

3. Tête selon la revendication 1, **caractérisée en ce que** l'organe d'absorption (13) est reçu dans deux logements (140, 122) ménagés respectivement dans la partie terminale (14) de la tête de battage (12) et dans une autre partie (200) de la tête de battage.

4. Tête selon l'une des revendications 1 à 3, **caractérisée en ce que** la tige centrale (5) est en outre pourvue d'un capteur d'accélération (6).

5. Tête selon la revendication 4, **caractérisée en ce que** deux capteurs de déformation (3, 4) sont disposés l'un derrière l'autre sur la tige centrale (5) qui est insérée en longueur, et **en ce que** la tige centrale (5) est insérée dans un corps creux (2) de la tête de mesure (1), recevant les capteurs (3, 4, 6) et limitant les perturbations électromagnétiques.

6. Tête de mesure selon l'une des revendications précédentes, **caractérisée en ce que** la première extrémité (50) de la tige centrale (5) est insérée dans un trou borgne (11) ménagé dans la tête de battage (12) et **en ce que** la deuxième extrémité (51) de la tige (5) comprend un trou borgne (7) dans lequel est insérée une extrémité (8) du train (9) de tiges (10).

7. Tête de mesure selon l'une des revendications précédentes, **caractérisée en ce que** la tige centrale (5) comprend un organe de liaison (51, 7) adapté pour relier la tête de mesure (1) à un train (9) de tiges (10) d'un pénétromètre dynamique.

8. Tête selon l'une des revendications précédentes, **caractérisée** en que la partie terminale (14) de la tête de battage (12) est configurée pour générer une onde de choc, notamment en forme de demi-lune.

9. Tête de mesure selon l'une des revendications précédentes, **caractérisée en ce qu'**elle est adaptée pour être reliée à un train (9) de tiges (10) dont la pointe (15) terminale est conique, l'angle (a) au sommet de la pointe (15) étant au moins de 60°.

10. Procédé de mesure à l'aide d'un pénétromètre dynamique équipé d'une telle tête de mesure (1) conforme à au moins une des revendications précédentes, **caractérisé en ce qu'**il comprend au moins les étapes suivantes :
- a) générer un choc (18) sur la partie terminale (14) de la tête de battage (12),
- b) collecter (30), à l'aide dudit au moins un capteur de déformation (3, 4), des signaux représentatifs des variations de la force résistante à la pointe et d'accélération lors de l'enfoncement de la pointe (15) dans le sol sous l'effet du choc sur la tête de battage,
- c) effectuer une filtration fréquentielle (31) des signaux de force résistante,
- d) effectuer une filtration temporelle (35) et une filtration fréquentielle (36) des signaux (34) d'accélération collectés,
- e) valider par comparaison les deux filtrations (35, 36) réalisées à l'étape d),
- f) considérer (32) les signaux obtenus aux étapes c) et d) comme des signaux corrigés,
- g) effectuer (41) un découplage des ondes sur les signaux corrigés,
- h) déterminer (42) la vitesse de propagation ou célérité de l'onde de compression dans le sol,
- i) reconstruire (43) les signaux relatifs au choc reçu à la pointe (15) du pénétromètre
- j) réaliser une courbe (44) représentative du comportement mécanique du sol, par exemple à l'aide des caractéristiques de résistance, de viscosité et de plasticité du sol,
- k) comparer ces caractéristiques avec celles de sols connus et en déduire le comportement mécanique du sol.

## Patentansprüche

1. Messkopf (1), der dazu bestimmt ist, ein dynamisches Penetrometer (19) auszustatten, und der dazu geeignet ist, mit einem Strang (9) von Stangen (10) verbunden zu werden, der mit einer Endspitze (15) ausgestattet ist, wobei der Messkopf (1) umfasst:
einen Schlagkopf (12), der dazu bestimmt ist, einen Stoß zur Übertragung auf den Strang (9) von Stangen (10) zu empfangen, wobei die Übertragung über den Rest des Messkopfes (1) erfolgt, und
eine zentrale Stange (5) zum Übertragen des Stoßes vom Schlagkopf auf den Strang von Stangen, wobei die zentrale Stange ein erstes Ende (50), das dem Schlagkopf zugewandt ist, und ein zweites Ende (51), das dem ersten Ende gegenüberliegt und das geeignet ist, mit dem Strang von Stangen zusammenzuwirken, aufweist, und wobei die zentrale Stange mit mindestens einem Deformationssensor (3, 4) versehen ist,
**dadurch gekennzeichnet, dass** er mindestens ein Absorptionselement (13) umfasst, das zwischen einem stoßempfangenden Endabschnitt (14) des Schlagkopfs (12) und dem zweiten Ende (51) der zentralen Stange (5) angeordnet ist und das geeignet ist, die an den Strang von Stangen übertragene Welle zu filtern, wenn der Endabschnitt des Schlagkopfes einen Stoß empfängt.

2. Messkopf nach Anspruch 1, **dadurch gekennzeichnet, dass** das Absorptionselement (13) aus einem Material hergestellt ist, das ein schlagfestes Polymer oder Polyethylen hoher Dichte enthält.

3. Messkopf nach Anspruch 1, **dadurch gekennzeichnet, dass** das Absorptionselement (13) von zwei Gehäusen (140, 122) aufgenommen wird, die jeweils in dem Endabschnitt (14) des Schlagkopfes (12) und in einem ein anderer Abschnitt (200) des Schlagkopfes angeordnet sind.

4. Messkopf nach einem der Ansprüche 1 bis 3, **dadurch gekennzeichnet, dass** die zentrale Stange (5) ferner mit einem Beschleunigungssensor (6) versehen ist.

5. Messkopf nach Anspruch 4, **dadurch gekennzeichnet, dass** die beiden Deformationssensoren (3, 4) hintereinander auf der längs eingesetzten zentralen Stange (5) angeordnet sind, und wobei die zentrale Stange (5) in einen Hohlkörper (2) des Messkopfes (1) eingesetzt ist, der die Sensoren (3, 4, 6) aufnimmt und elektromagnetische Störungen begrenzt.

6. Messkopf nach einem der vorhergehenden Ansprüche, **dadurch gekennzeichnet, dass** das erste Ende (50) der zentralen Stange (5) in ein im Messkopf ausgebildetes Sackloch (11) eingesetzt ist, und wobei das zweite Ende (51) der Stange (5) ein Sackloch (7) aufweist, in welches ein Ende (8) des Stranges (9) von Stangen (10) eingesetzt ist.

7. Messkopf nach einem der vorhergehenden Ansprüche, **dadurch gekennzeichnet, dass** die zentrale Stange (5) ein Verbindungelement (51, 7) umfasst, welches dafür geeignet ist, den Messkopf mit dem Strang (9) von Stangen (10) eines dynamischen Penetrometers zu verbinden.

8. Messkopf nach einem der vorhergehenden Ansprüche, **dadurch gekennzeichnet, dass** der Endabschnitt (14) des Messkopfs (12) ausgebildet ist, eine Schockwelle zu erzeugen, insbesondere in Form eines Halbmonds.

9. Messkopf nach einem der vorhergehenden Ansprüche, **dadurch gekennzeichnet, dass** er dafür geeignet ist, mit einem Strang (9) von Stangen (10) verbunden zu werden, dessen Endspitze konisch ist, wobei am oberen Ende der Winkel (α) mindestens 60° beträgt.

10. Messverfahren mittels eines dynamischen Penetrometers, welches mit einem solchen Messkopf gemäß mindestens einem der vorhergehenden Ansprüche ausgestattet ist, **dadurch gekennzeichnet, dass** es mindestens einen der folgenden Schritte umfasst:
- a) Erzeugen eines Stoßes (18) am Endabschnitt (14) des Schlagkopfes (12),
- b) Erfassen (30), mittels des besagten mindestens einen Deformationssensors (3, 4), von Signalen, welche repräsentativ für Schwankungen der Widerstandskraft an der Spitze und der Beschleunigung beim Versenken der Spitze (15) in den Boden unter dem Einwirkung des Stoßes auf den Schlagkopf sind,
- c) Ausführen einer Frequenzfilterung (31) von Widerstandskraftsignalen,
- d) Ausführen einer Zeitfilterung (35) und einer Frequenzfilterung (36) von erfassten Beschleunigungssignalen,
- e) Validieren der beiden im Schritt d) ausgeführten Filterungen (35, 36) durch Vergleich,
- f) Erachten (32) der in den Schritten c) und d) erhaltenen Signalen als korrigierte Signale,
- g) Ausführen (41) einer Entkopplung der Wellen in den korrigierten Signalen,
- h) Bestimmen (42) der Ausbreitungsgeschwindigkeit oder Schnelligkeit der Kompressionswelle im Boden,
- i) Rekonstruieren (43) der Signale bezüglich des an der Spitze (15) des Penetrometers empfangenen Stoßes,
- j) Erstellen einer Kurve (44), die repräsentativ für das mechanische Verhaltens des Boden ist, beispielsweise mittels Eigenschaften der Festigkeit, Viskosität oder Plastizität des Bodens,
- k) Vergleichen der Eigenschaften mit denen bekannter Böden und Ableiten eines des mechanischen Verhaltens des Bodens daraus.

## Claims

1. Measuring head (1), intended to be provided on a dynamic penetrometer (19) and suitable to be connected to a string (9) of rods (10) provided with an end tip (15), with this measuring head (1) comprising:
- a driving head (12) intended to receive an impact to be transmitted, via the rest of the measuring head (1), to the string (9) of rods (10), and
- a central rod (5) for transmitting the impact from the driving head to the rod string, said central rod has a first end (50), which is turned towards the driving head, and a second end (51), which is opposite the first end and which is suitable for engaging with the rod string, and said central rod is provided with at least one deformation sensor (3, 4),
**characterised in that** it comprises at least one absorption member (13), which is interposed between an end impact receiving portion (14) of the driving head (12) and the second end (51) of the central rod (5) and which is suitable for filtering the wave transmitted to the rod string when the end portion of the driving head receives an impact.

2. Head according to claim 1, **characterised in that** the absorption member (13) is made from a material comprising a high-impact polymer or high-density polyethylene.

3. Head according to claim 1, **characterised in that** the absorption member (13) is received in two housings (140, 122) arranged respectively in the end portion (14) of the driving head (12) and in another portion (200) of the driving head.

4. Head according to one of claims 1 to 3, **characterised in that** the central rod (5) is furthermore provided with an acceleration sensor (6).

5. Head according to claim 4, **characterised in that** two deformation sensors (3, 4) are arranged one behind the other on the central rod (5) which is inserted in length, and **in that** the central rod (5) is inserted into a hollow body (2) of the measuring head (1), receiving the sensors (3, 4, 6) and limiting the electromagnetic disturbances.

6. Measuring head according to one of the preceding claims, **characterised in that** the first end (50) of the central rod (5) is inserted into a blind hole (11) arranged in the driving head (12) and **in that** the second end (51) of the rod (5) comprises a blind hole (7) wherein is inserted an end (8) of the string (9) of rods (10).

7. Measuring head according to one of the preceding claims, **characterised in that** the central rod (5) comprises a connecting member (51, 7) suitable for connecting the measuring head (1) to a string (9) of rods (10) of a dynamic penetrometer.

8. Head according to one of the preceding claims, **characterised in that** end portion (14) of the driving head (12) is configured to generate a shockwave, in particular in the form of a half-moon.

9. Measuring head according to one of the preceding claims, **characterised in that** it is adapted to be connected to a string (9) of rods (10) of which the end tip (15) is tapered, with the angle (α) at the top of the tip (15) being at least 60°.

10. Method of measurement using a dynamic penetrometer provided with such a measuring head (1) in accordance with at least one of the preceding claims, **characterised in that** it comprises at least the following steps:
- a) generating an impact (18) on the end portion (14) of the driving head (12),
- b) collecting (30), using said at least one deformation sensor (3, 4), signals that are representative of the variations of the resistive force at the tip and for acceleration during the driving of the tip (15) in the ground under the effect of the impact on the driving head,
- c) carrying out a frequency filtering (31) of the resistive force signals,
- d) carrying out a temporal filtering (35) and a frequency filtering (36) of the collected acceleration signals (34),
- e) validating through comparison of the two filtrations (35, 36) carried out in step d),
- f) considering (32) the signals obtained in steps c) and d) as corrected signals,
- g) carrying out (41) a decoupling of the wavers on the corrected signals,
- h) determining (42) the speed of propagation or swiftness of the compression wave in the ground,
- i) reconstructing (43) the signals concerning the impact received at the tip (15) of the penetrometer
- j) creating a curve (44) that represents the mechanical behaviour of the ground, for example using the resistance characteristics, viscosity and plasticity of the ground,
- k) comparer these characteristics with those of known ground and in deducing the mechanical behaviour of the ground.
